# EUROPEAN PATENT SPECIFICATION

(11) **EP 1 784 486 B1**
(45) Date of publication and mention of the grant of the patent: **05.10.2011**
(21) Application number: 05762470.2
(22) Date of filing: 23.06.2005
(51) Int. Cl.: C12N 15/00, C12N 15/12

(54) **NERVE REGENERATION**
NERVENREGENERATION
REGENERATION DU NERF

(30) Priority: 23.06.2004 US 582627 P
(43) Date of publication of application: 16.05.2007
(73) Proprietor: TissueGene, Inc., Gaithersburg, MD 20877 (US)
(72) Inventor: KIM, Chang Hwan Dept Rehabilitation Medicine, Inchon (KR); LEE, Kwan Hee, Gaithersburg, MD 20877 (US)
(74) Representative: Fiener, Josef
(86) International application number: PCT/US2005/021993
(87) International publication number: WO 2006/002202

(56) References cited:
- WO-A-2005/068498
- WO-A1-03/059376
- US-A1- 2003 032 098
- US-A1- 2005 013 870
- PATRICK C W ET AL: "MURISTERONE A-INDUCED NERVE GROWTH FACTOR RELEASE FROM GENETICALLY ENGINEERED HUMAN DERMAL FIBROBLASTS FOR PERIPHERAL NERVE TISSUE ENGINEERING" TISSUE ENGINEERING, LARCHMONT, NY, US, vol. 7, no. 3, June 2001 (2001-06), pages 303-311, XP009037415 ISSN: 1076-3279
- PATRICK C W ET AL: "Dermal fibroblasts genetically engineered to release nerve growth factor." ANNALS OF PLASTIC SURGERY DEC 2001, vol. 47, no. 6, December 2001 (2001-12), pages 660-665, XP009084598 ISSN: 0148-7043
- WINN S R ET AL: "POLYMER-ENCAPSULATED CELLS GENETICALLY MODIFIED TO SECRETE HUMAN NERVE GROWTH FACTOR PROMOTE THE SURVIVAL OF AXOTOMIZED SEPTAL CHOLINERGIC NEURONS" PROCEEDINGS OF THE NATIONAL ACADEMY OF SCIENCES OF USA, NATIONAL ACADEMY OF SCIENCE, WASHINGTON, DC, US, vol. 91, no. 6, 6 March 1994 (1994-03-06), pages 2324-2328, XP001076846 ISSN: 0027-8424
- OKA N. ET AL.: 'Neuregulin is associated with nerve regeneration in axonal neuropathies' NEUROREPORT vol. 11, no. 17, 27 November 2000, pages 3673 - 3675, XP008081556
- HUIJBREGTS R. ET AL.: 'Hypertrophic neuropathies and malignant peripheral nerve sheath tumors in transgenic mice overexpressing glial growth factor beta3 in myelinating Schwann cells' JOURNAL OF NEUROSCIENCE vol. 23, no. 19, 13 August 2003, pages 7269 - 7280, XP003013566

## Description

### BACKGROUND OF THE INVENTION

**1. Field of the Invention:**

The present invention relates to prevention of nerve degeneration and promotion of nerve regeneration using cell based therapy.

**2. General Background and State of the Art:**

Numerous studies have been done on nerve regeneration for injured nerve. Generally, clinical surgical treatment for traumatic injury of peripheral nerve employs a complex series of steps involving first removing damaged tissues near the localized site of injury and providing an environment which enables regeneration of peripheral nerve (Kline, J Neurosurg, 1989,70: 166-174). The mechanics of the surgical procedure typically involve directly connecting or fusing the top and bottom of injured area (Kline and Judice, J Neurosurg, 1983, 58: 631-649). Additional surgical methods such as peripheral nerve transplantation (Millesi, Clin Orthop, 1988, 237: 36-42) as well as more conservative out-patient treatments such as maintaining electrical stimulation in order to generate muscular contraction which help inhibit degeneration of muscle-nerve junctions while waiting for voluntary regeneration of nerve (Kim et al., Korean Academy of Rehabilitation Medicine (Korean), 1999, 23: 893-8983; al-Amood et al., J Physiol (Lond), 1991, 441: 243-256). Finally, the majority of these procedures still involve extensive physical therapy regimens involving long-term and controlled exercise therapy in order to prevent muscular weakening and contraction and to promote collateral sprouting of nerve (Pyun et al., Korean Academy of Rehabilitation Medicine (Korean), 1999, 23; 1063-1075).

US 2003/032 098 A1 discloses the use of bone morphogenetic protein (BMP) for cartilage/bone repair, formation or maintenance by direct administration of the polypeptide or after transfecting fibroblast cells.

Patrick C. W., Tissue Engineering, vol. 7, no. 3, June 2001, pages 303-311, describes successful engineering of human dermal fibroblast to secrete NGF. Animal model was used to test for expression or secretion of NGF, fi. by intramuscular injection.

Frequently, mechanical orthopedic devices are used to protect joints and prevent damage of muscle and ligaments surrounding the affected area (Gravois, Physical Medicine and Rehabilitation, Massachusetts: Blackwell Science, 2000, pp432-433). In addition, various experimental techniques for nerve regeneration also involve the usage of biosynthetic tubes to connect nerves or are in combination with pharmaceuticals that collectively serve to reduce damage of nerve and stimulate regeneration of nerve (al-Amood et al., J Physiol (Lond), 1991, 441: 243-256; Dumitru, In: Dumitru, editor. Electrodiagnostic medicine, 1st ed, Philadelphia: Hanley & Belfus, 1995, pp341-384; Ebara and Nakayama, Spine, 2002, 16S: S10-S15; Horowitz, Muscle Nerve, 1989, 12: 314-322; Matzuk et al., Nature, 1995, 374: 360-363; Mengs, Arch Int Pharmacodyn Therp, 1984, 271 (2): 315-323).

Thus, there is a need for regenerating or preventing the degeneration of injured nerve and in particular peripheral nerve.

### SUMMARY OF THE INVENTION

The present invention is directed to a use as defined in claim 1.
The BMP may be BMP-2 or BMP-9. Further, the nerve may be peripheral nerve. The vector may be a viral vector. The vector may be a retroviral vector, adeno-associated Vital vector, adenoviral vector, or herpes viral vector. In addition, the population of cells may be stored prior to transplantation such as in 10% DMSO under liquid nitrogen.

### BRIEF DESCRIPTION OF THE DRAWINGS

The present invention will become more fully understood from the detailed description given herein below, and the accompanying drawings which are given by way of illustration only, and thus are not limitative of the present invention, and wherein;

FIGURES 1A-1B show a microscopic examination of rat sciatic nerve 2 weeks after injury in BMP-9 group, which shows very severe degeneration of axons and myelin sheath, and prominent vacuolated changes. FIGURES 1A-1B also show a significant infiltration of mononuclear inflammatory cells in the perineural soft tissue. (A; H&E stain, B; modified Trichrome stain 200 ×)

FIGURES 2A-2B show microscopic examination of rat sciatic nerve 4 weeks after injury in BMP-9 group, which shows that about 70% of the injured area is occupied by vacuolated change. Some myelin digestion chambers are noted and mild endoneural fibrosis is also noted. (A; H&E stain, B; modified Trichrome stain 200 ×)

FIGURES 3A-3C show microscopic examination of rat sciatic nerve 8 weeks after injury in BMP-9 group, which shows vacuolated changes in about 50% of the axons. Severe mononuclear inflammatory cells are infiltrated in epineural space. Focal hemorrhage is also present in the nerve bundle. (A; H&E stain× 200, B & C; modified Trichrome stains 200 × & 100 ×, respectively)

FIGURES 4A-4B show microscopic examination of rat sciatic nerve 8 weeks after injury in BMP-9 group, which shows vacuolated changes in about 33% of the axons. Some mononuclear inflammatory cells remain in the epineural space. (A & B; modified Trichrome stains 200 ×)

FIGURES 5A-5B show microscopic examination of rat sciatic nerve 2 weeks after injury in BMP-2 group, which shows vacuolar changes in most of the axons. Axonal atrophy and demyelination is noted in the center. (A & B; modified Trichrome stains 100 × & 200 ×, respectively)

FIGURES 6A-6B show microscopic examination of rat sciatic nerve 4 weeks after injury in BMP-2 group, which shows vacuolar changes in about half of the axons. Demyelination is noted with mild endoneural fibrosis. (A & B; modified Trichrome stains 100 × & 200 ×, respectively)

FIGURES 7A-7B show microscopic examination of rat sciatic nerve 8 weeks after injury in BMP-2 group, which shows vacuolated changes in about a half of the axons. Some mononuclear inflammatory cells are remaining in the epineural space. (A & B; modified Trichrome stains 100 × & 200 ×, respectively)

FIGURES 8A-8B show microscopic examination of rat sciatic nerve 2 weeks after injury in sham group, which shows that almost all axons are degenerated and mononuclear inflammatory cells are infiltrated in the periphery of the axons. Epineural fibrosis is also noted (A & B; modified Trichrome stains 100 × & 200 ×, respectively).

FIGURES 9A-9B show microscopic examination of rat sciatic nerve 4 weeks after injury in sham group, which shows that about 33% of the axons are degenerated and vacuolar changes are prominent. (A & B; modified Trichrome stains 100 × & 200 ×, respectively).

FIGURES 10A-10B show microscopic examination of rat sciatic nerve 8 weeks after injury in sham group, which shows that about 10~20% of axons are degenerated, and vacuolar changes are prominent. (A & B; modified Trichrome stains 100 × & 200 ×, respectively).

### DETAILED DESCRIPTION OF THE PREFERRED EMBODIMENTS

In the present application, "a" and "an" are used to refer to both single and a plurality of objects.

As used herein, the term "connective tissue cell" or "cell of a connective tissue" include cells that are found in the connective tissue, such as fibroblasts, cartilage cells (chondrocytes), and bone cells (osteoblasts/ osteocytes), which secrete collagenous extracellular matrix, as well as fat cells (adipocytes) and smooth muscle cells. Preferably, the connective tissue cells are fibroblasts, cartilage cells, and bone cells. More preferably, the connective tissue cells are fibroblast cells. Connective tissue cells also include mesenchymal cells, which are also known as immature fibroblasts. It will be recognized that the invention can be practiced with a mixed culture of connective tissue cells, as well as cells of a single type.

As used herein, injection of cells "near" an injured nerve is meant that area which is close enough between the injection site and the injured nerve to effect an efficacious outcome of regenerating nerve at the injured site. Therefore, the injection of cells near an injured nerve includes at the site of injury or anywhere close enough for the injected cells to express the effective polypeptide and the polypeptides are allowed to directly or indirectly effect the nerve regenerating outcome.

As used herein, a "promoter" can be any sequence of DNA that is active, and controls transcription in an eucaryotic cell. The promoter may be active in either or both eucaryotic and procaryotic cells. Preferably, the promoter is active in mammalian cells. The promoter may be constitutively expressed or inducible. Preferably, the promoter is inducible. Preferably, the promoter is inducible by an external stimulus. More preferably, the promoter is inducible by hormones or metals. Still more preferably, the promoter is inducible by heavy metals. Most preferably, the promoter is a metallothionein gene promoter. Likewise, "enhancer elements", which also control transcription, can be inserted into the DNA vector construct, and used with the construct of the present invention to enhance the expression of the gene of interest.

As used herein, "selectable marker" includes a gene product that is expressed by a cell that stably maintains the introduced DNA, and causes the cell to express an altered phenotype such as morphological transformation, or an enzymatic activity. Isolation of cells that express a transfected gene is achieved by introduction into the same cells a second gene that encodes a selectable marker, such as one having an enzymatic activity that confers resistance to an antibiotic or other drug. Examples of selectable markers include, but are not limited to, thymidine kinase, dihydrofolate reductase, aminoglycoside phosphotransferase, which confers resistance to aminoglycoside antibiotics such as kanamycin, neomycin and geneticin, hygromycin B phosphotransferase, xanthine-guanine phosphoribosyl transferase, CAD (a single protein that possesses the first three enzymatic activities of *de novo* uridine biosynthesis - carbamyl phosphate synthetase, aspartate transcarbamylase and dihydroorotase), adenosine deaminase, and asparagine synthetase (Sambrook et al. Molecular Cloning, Chapter 16, 1989).

As used herein, the "transforming growth factor-β (TGF-β) superfamily" encompasses a group of structurally related proteins, which affect a wide range of differentiation processes during embryonic development. The family includes, Mullerian inhibiting substance (MIS), which is required for normal male sex development (Behringer, et al., Nature, 345:167, 1990), Drosophila decapentaplegic (DPP) gene product, which is required for dorsal-ventral axis formation and morphogenesis of the imaginal disks (Padgett, et al., Nature, 325:81-84, 1987), the Xenopus Vg-1 gene product, which localizes to the vegetal pole of eggs (Weeks, et al., Cell, 51:861-867, 1987), the activins (Mason, et al., Biochem, Biophys. Res. Commun., 135:957-964, 1986), which can induce the formation of mesoderm and anterior structures in Xenopus embryos (Thomsen, et al., Cell, 63:485, 1990), and the bone morphogenetic proteins (BMP's, such as BMP-2, 3, 4, 5, 6 and 7, osteogenin, OP-1) which can induce *de novo* cartilage and bone formation (Sampath, et al., J. Biol. Chem., 265:13198, 1990). The TGF-β gene products can influence a variety of differentiation processes, including adipogenesis, myogenesis, chondrogenesis, hematopoiesis, and epithelial cell differentiation (for a review, see Massague, Cell 49:437,1987).

The proteins of the TGF-β family are initially synthesized as a large precursor protein, which subsequently undergoes proteolytic cleavage at a cluster of basic residues approximately 110-140 amino acids from the C-terminus. The C-terminal regions of the proteins are all structurally related and the different family members can be classified into distinct subgroups based on the extent of their homology. Although the homologies within particular subgroups range from 70% to 90% amino acid sequence identity, the homologies between subgroups are significantly lower, generally ranging from only 20% to 50%. In each case, the active species appears to be a disulfide-linked dimer of C-terminal fragments. For most of the family members that have been studied, the homodimeric species has been found to be biologically active, but for other family members, like the inhibins (Ung, et al., Nature, 321:779, 1986) and the TGF-β's (Cheifetz, et al., Cell, 48:409, 1987), heterodimers have also been detected, and these appear to have different biological properties than the respective homodimers.

Members of the superfamily of TGF-β genes include TGF-β3, TGF-β2, TGF-β4 (chicken), TGF-β1, TGF-β5 (*Xenopus*), BMP-2, BMP-3, BMP-4, BMP-5, BMP-6, OP-1/BMP-7, BMP-8, BMP-9, *Drosophila* 60A, *Drosophila* DPP, Vgr1, GDF-1, *Xenopus* Vgf, Inhibin-βA, Inhibin-βB, Inhibin-α, and MIS. Many of these genes are discussed in Massague, Ann. Rev. Biochem. 67:753-791, 1998.

Preferably, the member of the superfamily of TGF-β genes is TGF-β. More preferably, the member is TGF-β1, TGF-β2, TGF-β3, BMP-2, BMP-3, BMP-4, BMP-5, BMP-6, BMP-7, BMP-8, or BMP-9.

It is understood that in describing a protein by its designated name as above, the protein is not limited to the exact sequence of the wild-type. Variations on the sequence of the protein is acceptable such that another polypeptide sequence, which exhibits substantially the same activity as the protein in terms of function is encompassed thereby.

**Nerve Tissue**

Nervous tissue derives from the embryonic ectoderm under the influence of the notochord. The ectoderm is induced to form a thickened neural plate that then differentiates and the ends eventually fuse to form the neural tube from which all of the central nervous system derives. The central nervous system consists of the brain, cranial nerves and spinal cord. The peripheral nervous system derives from cells next to the neural groove called the neural crest.

Nerve tissue is distributed throughout the body in a complex integrated communications network. Nerve cells (neurons) communicate with other neurons in circuits ranging form very simple to very complex higher-order circuits. Neurons do the actual message transmission and integration while other nervous tissue cells called glial cells assist neurons by support, protection, defense and nutrition of the neurons. There are about 10 times more glial cells than neurons in the brain, Glial cells create the microenvironment needed for neuronal function and sometimes they assist in neural processing and activity. Neurons are excitable cells. This means that when properly stimulated, an action potential can be initiated that may be propagated over the cell membrane to transmit information to distant cells. Neurons are independent functional units responsible for the reception, transmission and processing of stimuli.

In general, neurons consist of three parts; the cell body, where the nucleus and cellular organelles are located; dendrites, which are processes extending from the cell body that receive stimuli from the environment or other neurons; and the axon, which is a long single process extending from the cell body for the transmission of nerve impulses to other cells. The axon usually branches at its distal end and each branch terminating on another cell has a bulbous end. The interaction of the end bulb with the adjacent cell forms a structure called a synapse. Synapses are specialized to receive a signal and convert it into an electrical potential.

Most neurons found in the human body are multipolar, meaning they have more than two cell processes with only one being an axon and the remaining processes being dendrites. Bipolar neurons of the retina or olfactory mucosa have one dendritic process and an axon coming off the cell body. Pseudounipolar neurons found in the spinal cord ganglia enable sensory impulses picked up by the dendrites to travel directly to the axon without passing through the cell body. Neurons may also be classified according to function. Sensory neurons are involved in the reception and transmission of sensory stimuli. Motor neurons send impulses to control muscles and glands. Other neurons, intemeurons, act as go-betweens between neurons as part of functional networks.

Synapses are specialized functional cell junctions to propagate cellular signals. Most synapses are chemical synapses where vesicles in the presynaptic terminal contain a chemical messenger that is released to the synaptic cleft when the presynaptic membrane is stimulated. The chemical messenger diffuses across the synaptic cleft to bind to receptors in the postsynaptic membrane. This induces a change in the polarization state of the postsynaptic membrane effecting cellular action. A special type of synapse is the neuromuscular junction. More than 35 neurotransmitters are known and most are small molecules (nitric oxide, acetylcholine), catecholamines (norepinephrine, serotonin), or neuroactive peptides (endorphin, vasopressin). Once used, the neurotransmitters are removed quickly by enzymatic breakdown, diffusion or endocytosis by the presynaptic cell.

Some neurons are wrapped in an insulating material called myelin. This lipid rich material is formed by glial cells: Schwann cells in the peripheral nervous system and by oligodendrocytes in the central nervous system. The insulation enables faster nerve conduction by reducing the membrane surface area that must be depolarized. In myelinated neurons the nerve impulse jumps from one unmyelinated segment to another over the length of the axon. It is the myelin sheath and lack of neuron cell bodies within the tissue that makes some nervous tissue appear white as in the large peripheral nerves and white matter of the brain. Other glial cells, called astrocytes, are involved in structural integrity, neuronal nutrition and maintaining the microenvironment of nervous tissue. Astrocytes, are in direct communication with one another via gap junctions and can affect the survival of neurons in their care by the regulation of the the local environment. Ependymal cells line spinal cord and the ventricles of the brain and secrete the cerebrospinal fluid. Other small glial cells, called microglia, are phagocytic cells that are involved with inflammation and repair in the adult central nervous system.

Nervous tissue is an excitable tissue that is capable of receiving and transmitting electrical impulses. The central cell type is called a neuron. Neurons usually have a cell body, dendrites that receive inputs, and an axon that transmits electrical potentials.

Neurons may be classified as sensory, motor, secretory or association neurons. They are often classified by conduction speed, diameter and the presence or absence of specialized lipoprotein insulation called myelin. Type A fibers are myelinated and can conduct impulses at 12 -120 m/sec. Type B are also myelinated fibers but they only transmit impulses at 3-5 m/sec. Type C fibers are unmyelinated, small in diameter and very slow (2.5 m/sec). An example of a Type A fiber is a motor neuron innervating the gastrocnemius. An autonomic preganglionic efferent neuron is an example of a Type B fiber and a sensory neuron carrying information about diffuse pain is an example of a slow Type C fiber.

Sensory neurons are adapted to detect certain types of information from the environment. These include mechanoreceptors sensing things like pressure or stretch, thermoreceptors, photoreceptors in the retina, and chemoreceptors such as the taste bud or those for olfaction. Association neurons, or intemeurons are usually found in the spinal cord and brain where they connect sensory afferent neurons to efferent motor or secretory neurons.

Neurons communicate with one another via a structure called the synapse. An axon ends in one or more terminal buttons that contain numerous small vesicles. These small vesicles are filled with chemical substances called neurotransmitters. Acetylcholine is most often the neurotransmitter at the synapse although other chemicals like norepinephrine, serotonin and GABA may be used dependent on the neuron. When an impulse travels down the axon and reaches the terminal buttons the vesicles fuse with the neuronal membrane and the neurotransmitter is released. The chemical then diffuses across the narrow synaptic cleft to specific receptors for the chemical on the postsynaptic membrane of the receiving neuron.

The interaction of the neurotransmitter with the receptor causes a change in the membrane potential that may induce a new impulse postsynaptic neuron. The enzyme acetylcholinesterase is present in synapse to break down acetycholine and terminate the stimulus. Other neurotransmitters are either broken down or taken back up into the presynaptic neuron to terminate the stimulus.

In the central nervous system many neurons may converge on a single neuron. When each of the presynaptic neurons releases neurotransmitter into its synapse with the postsynaptic neuron, local membrane potentials occur that are integrated and summed. These incoming signals may be inhibitory or stimulatory. If the resulting summed membrane potential reaches the minimum threshold for that neuron, then an action potential will be initiated.

Action potentials travel in one direction away from the cell body by saltatory conduction. The fastest neurons are covered in myelin sheaths arranged in discreet segments separated by nodes of naked neuronal membrane called nodes of Ranvier. In saltatory conduction, the electrical potential jumps from node to node, thereby reducing the membrane area involved in conduction of the action potential and speeding up conduction.

Non-neural cells found in the nervous system are called glial cells. Astrocytes are the most numerous and provide support and nourishment of neurons. Microglia are small phagocytic cells specific to neural tissue. Cells that line the ventricular system and central canal of the spinal cord and make cerebrospinal fluid are called ependymal cells. In the central nervous system, an oligodendrocyte forms segments of the myelin sheaths of multiple neurons. In the peripheral nervous system, each segment of the myelin sheath is made by a single Schwann cell.

**Central nervous system**

The central nervous system (CNS) consists of the brain and spinal cord. The meninges (dura mater, arachnoid and pia mater) protect and nourish the CNS in addition to the protection afforded by the bony skull and vertebrae. Cerebrospinal fluid is found in the the subarachnoid space, central canal of the spinal column and the ventricles of the brain. The pia mater is the innermost layer and is adherant to the nervous tissue. Between the pia mater and the dura mater lies the arachnoid layer. The tough fibrous dura mater lies just beneath the skull.

The brain can be divided into 3 basic areas of the forebrain, midbrain, and brain stem. The forebrain includes the thalamus, hypothalamus, basal ganglia, and cerebrum. The cerebrum is responsible for conscious thought, interpretation of sensations, all voluntary movements, mental faculties, and the emotions.

Cerebral tissue can be divided into structural and functional areas. The surface of the cerebrum is convoluted into gyri (ridges) and sulci (grooves). The cortical sensory and motor areas can be mapped to the post central gyrus and central sulcus, respectively. The sensory area receives sensory info from the opposite side of the body that is projected after thalamic processing. Those parts of the body with more sensory nerve endings are represented by more cortical sensory area. The motor area controls voluntary muscle movements of the contralateral body parts but the association areas are important for the initiation of movement.

The cerebrum is the largest part of the brain and is divided into two hemispheres, right and left, having several lobes. The frontal lobe contains the motor area, Broca's speech area, association areas, and functions in intelligence and behavior. The parietal lobe contains sensory areas and function in feeling and hearing. Primary visual association areas are located in the occipital lobe and the temporal lobe contains areas for auditory association, smell and memory storage.

The thalamus is located between the cerebral cortex and brainstem. All sensory input except the sense of smell is processed here before being projected to other areas of the brain. The hypothalamus is located beneath the thalamus and is responsible for processing internal stimuli and the maintenance of the internal environment. Moment by moment unconscious control of blood pressure, temperature, heart rate, respiration, water metabolism, osmolality, hunger, and neuroendocrine activities are handled here. Nuclei of the neuroendocrine cells that release oxytocin and ADH from the posterior pituitary are located in the hypothalamus.

The basal ganglia (caudate nucleus, globus palladus, substantia nigra, subthalamic nucleus, red nucleus) are groups of neurons embedded within each hemisphere of the cerebrum. They are involved in the control of complex motor control, information processing and unconscious gross intentional movements.

The brainstem includes the medulla oblongata and pons. The medulla oblongata contains important functional areas and relay centers for the control of respiration, cardiac and vasomotor reflexes. The pons contains the pneumotaxic center which is involved in the regulation of respiration.

The cerebellum lies above the brainstem and uses sensory information processed elsewhere about the position of the body, movement, posture and equilibrium. Movements are not initiated in the cerebellum but it is necessary for coordinated movement.

**Peripheral nervous system**

The peripheral nervous system includes nerves, ganglia, spinal and cranial nerves located outside the brain and spinal cord. The twelve cranial nerves arise from nuclei located in the brainstem and travel to specific locations carrying impulses to control various autonomic functions like smell, vision, salivation, heart rate and cutaneous sensation. Cranial nerves are often mixed in that they carry sensory and motor components but they may have only motor or sensory fibers. The following table lists the cranial nerves and their functions.

**Table 1 - Cranial Nerves**

| **Number** | **Name** | **Function** |
|---|---|---|
| I | Olfactory | Sense of smell |
| II | Optic | Vision |
| III | Oculomotor | Motor control of some eye muscles and eyelid |
| IV | Trochlear | Motor control of some eye muscles |
| V | Trigeminal | Chewing muscles and some facial sensation |
| VI | Abducent | Motor control of some eye muscles |
| VII | Facial | Motor control of facial muscles, salivation. Taste and cutaneous sensations. |
| VIII | Acoustic | Equilibration, static sense and hearing |
| IX | Glossopharyngeal | Salivation, sensations of skin, taste and viscera |
| X | Vagus | Motor control of the heart and viscera, sensation from the thorax, pharynx and abdominal viscera |
| XI | Accessory | Motor impulses to the pharynx and shoulder |
| XII | Hypoglossal | Motor control of the tongue, some skeletal muscles, some viscera, sensation from skin and viscera |

The sensory division of the peripheral nervous system takes input from various types of receptors, processes it and sends to the central nervous system. Sensory input can come from internal sources as in proprioception (sense of position of the joints and muscles) or external sources as in the sensation of pressure or heat on the skin. Areas of the skin innervated by specific spinal nerves are called dermatomes. Afferent fibers collect sensory input and travel up the spinal cord, converge in the thalamus, and end finally on the sensory cortex of the cerebrum. Those areas with more sensory receptors, i.e. the fingertips or lips, correspond to a larger area on the sensory cortex of the brain. Fibers carrying proprioceptive information are dispersed to the cerebellum as well. Almost all sensory systems transmit impulses to parts of the thalamus. The cerebral cortex is involved in conscious perception and interpretation of sensory stimuli.

Motor inputs to muscles and glands occur via the autonomic and somatic efferent systems. CNS innervation of the joints, tendons and muscles travel via the somatic efferent system. Some muscular responses are handled via spinal reflexes. An example of this is the withdrawal reflex seen when the finger contacts a hot stove. The movement to remove the finger occurs via a simple spinal reflex long before the sensation of pain reaches the brain. Clearly this is protective mechanism to avoid further injury. Motor inputs to glands and smooth muscle usually occur via the autonomic system.

Most organs receive input from both branches of the autonomic nervous system. One branch will generally be excitatory while the other is inhibitory in that organ or tissue. The sympathetic branch of the autonomic system acts to prepare the body for physiologic stress. Stimulation of the sympathetic branch is like stepping on the gas in that the body prepares to run or fight in response. Effects such as an increased heart rate, dilation of airways and mobilization of glucose from glycogen stores are seen. Sympathetic nerves arise from the 1^{st} thoracic to the 4^{th} lumbar vertebra. They have a short preganglionic neuron that ends in one of the chain ganglia that lie along the spinal column. Acetylcholine is the neurotransmitter at the synapse with the long postganglionic neuron which then travels to the target tissue where norepinephrine is released at the majority of sympathetic nerve endings. A few sympathetic post ganglionic neurons, such as those innervating sweat glands or skeletal muscle vasculature, release acetylcholine.

The parasympathetic branch acts to counterbalance the sympathetic branch via neurons that arise from the cranial and sacral regions of the CNS. For instance, parasympathetic stimulation constricts airways and decreases heart rate. It regulates resting activities such as digestion, micturation and erection. Long preganglionic neurons release acetylcholine at synapses close to the end organ. Short postganglionic neurons also release acetylcholine on the effector tissue.

**Regeneration of Damaged Nerve Cells By Myelin Producing Cells**

In one aspect of the invention, in order for nerve degeneration to occur two events must occur. First, the regeneration factor producing cells must be viable as well as the nerve cells that are receiving the benefit of the therapy. The second is that myelination of the neurons must occur. In this regard, a variety of different cell types may be used to effectuate this phenotype. For example, a mixture of cell viability inducing cells and myelin producing cells may be used together to regenerate the nerve.

It is especially contemplated that myelination of the nerve cells is especially applicable to healing spinal cord damage by regenerating the damaged nerves. In this regard, a protein such as axonal neuregulin-1 (Nrg1) (Michailov et al., Science, 304, 700-703, 4/30/2004), which expression induces myelination may be used and expressed in a nerve cell, such as but not limited to Schwann cell, alone or in conjunction with another same cell or different cell type, such as a connective tissue cell including but not limited to fibroblast cell that may express Nrg1.

**Effect Of BMP On Regeneration of Nerve and Protection From Nerve Degeneneration**

One particular and exemplified aspect of the invention is directed to determining the effect of the functional proteins of BMP-2 and BMP-9 on regeneration after nerve injury in rat. Thirty six Sprague-Dawley strain (weight, 400±10 g) rats were used, which were divided into 3 groups (control, BMP-2, and BMP-9). It is noted here that by BMP-2 or BMP-9 group, it refers to the animal group that will be injected with cells expressing BMP-2 or BMP-9 after the nerve damage is done to these uninjected Sprague-Dawley rats. Using a haemostatic forceps, crushing injuries were made on sciatic nerves. In the control group, only buffer with non-modified fibroblasts were injected. In BMP-2 and BMP-9 groups, buffer with gene-modified fibroblasts were injected (BMP-2: BMP-2 secreting fibroblast, BMP-9: BMP-9 secreting fibroblast). After completion of injection, effects on regeneration were evaluated by 1) sciatic nerve motor conduction study recorded at the gastro-soleus muscles, and 2) histologic studies on sciatic nerve with hematoxylin-eosin, and modified Trichrome stain.

The nerve conduction study revealed significantly short latencies in BMP-2 and BMP-9 groups compared with the control group at 1 week, and amplitudes were subsequently shown to be greater in the BMP-9 group in weeks 4 and 6, compared with other groups (p<0.05). In week 8, there was no apparent difference of latency among all groups, but the amplitudes were greater in the BMP-9 group.

In the histological study, all groups showed severe degeneration (by means of exhibiting an inflammatory response, loss of axons and vacuolar changes) by week 2. In the control group, the degeneration persisted in weeks 4 and 8, but in BMP-2 group, the size of inflammation and vacuolar changes were significantly reduced, and of which only half of the group showed these findings. In BMP-9 group, these changes were even further reduced with only one third of them showing loss of axon and vacuolar changes, and inflammation was very mild. The effects of these functional proteins on nerve regeneration after crush injury were significant in these rats. These results show the regeneration of nerve after nerve injury, including peripheral nerve injury.

TGF-β, activins, and BMP are proteins involved in cell differentiation, growth and organ formation during development. BMP is a member of the TGF-β superfamily along with growth/differentiation factors (GDF), osteogenic proteins (OPs), and Mullerian inhibiting substance/anti-Mullerian Hormone (MIS/AMH) (Ebara and Nakayama, Spine, 2002, 16S: S10-S 15). Historically, in 1965, Urist (Urist, MR: Bone, formation by autoinduction, Science, 1965, 150(698): 893-899) observed the phenomenon of embryonic ossification and other processes similar to endochondral ossification after inserting demineralized bone matrix into the muscle of both rodents and rabbits. After implantation, undifferentiated mesenchymal cells migrated to the inserted bone tissues by chemotaxis, followed by mitosis and condensation. Chondroblasts, which are derived from mesenchymal cells, then secreted extracellular matrix, which enabled formation of the cartilage template. This extracellular matrix is vascularized through hematopoietic and endothelial cells. Osteoblast and osteoclast began to appear locally and absorbed cartilage was transformed into bone tissues. After 21 days, ossicle with marrow core of bone was formed (Wang et al., Proc Nat Acad Sci USA, 1988, 85: 9484-9488). The component, which was associated with this changing process from the demineralized bone matrix, was described as bone morphogenic protein (BMP).

In 1988, Wang et al. (Wang et al., Proc Nat Acad Sci USA, 1988, 85: 9484-9488) isolated three polypeptides with the molecular weights of 16 kDa, 18 kDa, and 30 kDa each from bovine bones. Wozney et al. (Woozney, Mol Rep Dev, 1992, 32: 160-167) later identified human RNAs and corresponding DNAs using these polypeptides as probes. Follow up studies have revealed the presence of at least 16 endogenous BMP's (Wozney and Rosen, Clin Orthop, 1998, 346: 26-37).

Except for BMP1 (procollagen C-protease), they are all members of transforming growth factor (TGF)- β gene superfamily (Wozney and Rosen, Clin Orthop, 1998, 346: 26-37). Structurally, BMPs are produced as a large precursor form composed of a 15-25 amino acid signal peptide, a prodomain with 50-375 amino acids, and mature terminal carboxyl terminal with 100-125 amino acids. The latter has well-conserved 7 cysteine residues, enabling peptide dimerization after the carboxy terminal region is cleaved from the precursor by proteolytic processing (Croteau et al., 1999; 22: 686-695). Each active, mature BMP protein exists as a disulfide-linked homodimer composed of the same monomers or disulfide-linked heterodimer composed of two different types of monomers (Sampath et al., J Biol Chem, 1990, 265: 13198-13250). Interestingly, the dimerization of the protein has been linked to its activity, and as such the heterodimers, BMP2 and BMP7 have been shown to be stronger morphogens than the homodimer composed of the same monomer (Kawabata et al., Cytokine Growth Factor Rev, 1998, 9: 49-61; Sampath et al., J Biol Chem, 1990, 265: 13198-13250).

In order to capitalize on the biological activity of BMP, it is necessary to understand the regulation of BMP gene expression and the mechanism of BMP dimerization in the cell. Although not much is known about the gene expression of BMP, it is known that it may be regulated by basic helix-loop-helix (bHLH) protein (Ebara et al., Biochem Biophys Res Commun, 1997, 240: 136-141). This bHLH protein is composed of 3 domains, with two outer domains acting as positive transcriptional activators and the center domain acting as a negative regulator. Among these domains, the E-box (the DNA Sequence ranging from 246-265bp) is recognized by the USF transcription factor and has an important role in regulating the expresison of BMP in the mouse. BMP has also been implicated in the regulation of the cell death pathway.

The biological activities of BMP are tightly regulated at various timepoints beyond the transcriptional level, and even extracellularly. It is thought that outside of the cells, receptors of BMP serving as inhibitory proteins, easily react with BMPs and in response to the increased activity of BMP, may elicit enhanced production of negative feedback signals ultimately leading to their regulation (Ebara and Nakayama, Spine, 2002, 16S: S10-S15). Inside, the cells are regulated both by signal transduction and by repressive Smad proteins, meaning that BMP is able to upregulate expression of repressive Smad proteins (Ebara and Nakayama, Spine, 2002, 16S: S10-S15).

At the extracellular level, the cells are controlled by BMP binding proteins such as noggin and chondrin, which inhibit binding of BMP to the cell receptor. Twisted gastrulation (Tsg) enhances the function of chondrin (Ebara and Nakayama, Spine, 2002, 16S: S10-S15). Follisatin binds to OP-1/BMP-7 and BMP-4 proteins and represses BMP. (Matzuk et al., Nature, 1995, 374: 360-363).

**Receptor for BMP**

BMP binds to two different types (type I and II) of serine-threonine kinase receptors. Two type I receptors and one type II receptor have been confirmed in mammals (Kawabata et al., Cytokine Growth Factor Rev, 1998, 9: 49-61). In mammals, the type I receptor has isoforms A and B and although they are structurally similar, they show different behaviour in their activation of the Smad proteins (Imamura et al., Nature, 1997, 389: 549-551). For signal transduction, type I and II receptors need to form a complex. Type I receptor is activated by type II receptor and the signal is transduced in cells by the type I receptor. The signal in the cells is transduced by Smad proteins. Smad1, Smad5 and Smad8 belong to the same structure and transduce the signal from BMP. Smad2 and Smad3 transduce signals from TGF-β and activin. These Smads form heteromeric complex and translocate into the nucleus to activate various genes. Smad6, Tob, Ski and Smurfl are involved in negative regulation of these genes. Among these, Smad6 represses the transcription of BMP and also has a role in negative feedback of BMP signaling pathway (Bai et al., J Biol Chem, 2000, 275: 8267-8270). Tob is a member of antiproliferative protein family and is involved in negative regulation of BMP/Smad signal (Yoshida et al., Cell, 2000, 103: 1085-1097). Ski oncoprotein represses the expression of BMP-signaling and BMP-responsive genes, and represses the activation of BMP by directly reacting with Smad complex, which is a characteristic of BMP (Wang et al., Proc Natl Acad Sci USA, 2000, 97: 14394-14399). Smurfl belongs to Hect family of ubiquitin ligase and inhibits signal transduction of BMP by selectively binding with receptor-regulated Smad (Zuh et al., Nature, 1999, 400: 687-693).

Further research on the function of the BMP family of proteins is ongoing and it seems that they are involved in the scheme of basic body formation including nerve system (Farkas et al., J Neurosci, 1999, 92: 227-235), eyes (Mohans et al., Invest Ophthalmol Vis Sci, 1998, 39: 2626-2636), lung, kidney, prostate, reproductive organ and hair follicles during the embryonic stage. For example, the formation of fingers and spaces between fingers has been reported to be due to apoptosis of cells between fingers caused by BMP (Zou and Niswander, Science, 1996, 272: 738).

BMP is involved in formation, differentiation and healing of skeletal system during the embryonic stage. In the skeletal system after birth, BMP is present in the collagen of brain stroma, periosteum cell and mesenchymal cell of matrix filled with blood forming elements. BMP also has been isolated from osteosarcoma and chondrosarcoma (Lianjia and Yan, Clin Orthop, 1990, 257: 249-256). After fracture, BMP is diffused in the absorbed bone matrix, activates osteoprogenitor cells, and in turn produces more BMP. Distribution of BMP depends on the time of the treatment and location of the fracture and may be further complicated due to reciprocal reactions. BMP research has also been carried out in a variety of other tissues to study their protective or regenerative effect, and have been demonstrated to have a protective effect on the function of heart muscle in ischemia and repurfusion of heart muscle (Lefer et al., J Mol Cell Cardiol, 1992, 24: 585-593), on the extended nervous system in experiments inducing cerebral ishemia after injecting BMP into abdominal cavity and regenerative effects on damaged kidneys (Ripamonti and Duneas, Plast Reconstr Surg, 1998, 101: 227-239).

The present application shows that BMP, which is involved in differentiation of peripheral nerve, advantageously heals damaged nerve when compared to the healing process of untreated groups.

***In vivo* and *Ex vivo* Methods**

The present invention is not limited to *ex vivo* methods of gene delivery to the site of intereset. *In vivo* methods are also contemplated. It is also to be noted that any vector may be used so long as it is able to express the desired protein within the site of injection. The vector may be a plasmid or any number of viral vectors so long as it is usefully employed to express the gene of interest.

### EXAMPLES

**EXAMPLE I -MATERIALS AND METHODS**

**Materials**

Mature Sprague-Dawley strain rats (16~18 weeks old) with weight of 400±10 g have been used for this study. The reason for using mature rats is because it is easier to observe changes due to nerve regeneration and physiological changes compared with rats in growing stage.

**Methods**

It is technically difficult to inject functional protein BMP into peripheral nerves. Thus, in this study, rat fibroblasts producing BMP2 and BMP9 were directly transfected into the peripheral nerves so that BMP2 and BMP9 are secreted locally. Total of 36 rats were divided into 3 groups. Each group consisted of 12 rats with injured nerves. The first group was a control group with rats whose injured nerves were treated with non-modified fibroblast without transgene. The second group (BMP2 group) was composed of rats with injured nerves, which were treated with gene-modified fibroblast with BMP-2 transgene, and the third group (BMP9 group) was composed of rats with injured nerves, which were treated with gene-modified fibroblast with BMP-9 transgene. Histological examination of tissues was performed 2, 4, and 8 weeks after the treatment by sacrificing 2 rats from each group and harvesting sciatic nerves from both limbs. Nerve motor conduction study was also performed with sciatic nerves from limbs by setting the original base-line value before the experiment and measuring nerve conduction every other week up to 8 weeks after the surgery.

**Nerve Injury**

The white rat was anesthetized by injecting 4% chloral hydrate in a concentration of 300 mg/Kg into abdominal cavity. Hair was removed from the posterior and femoral regions of the right limb before being fixed in prone position. After sterilizing the femoral region with potadine and 70% alcohol, about 1~1.5 cm of epidermis around the central part of the femoral region was vertically incised and femoral biceps were pulled outward to expose sciatic nerve. Nerve injuries were made according to DeKoning et al. (De Koning et al., J Neurol Sci, 1986, 74: 237-246) by incising the skin between the thigh and total knee joint to 2~2.5 cm in length and exfoliating the posterior and total knee joint muscles to expose sciatic nerve and then injuring the nerve exposed at sciatic herniation by crushing with haemostatic forceps (Crile, 15 cm) for 30 seconds. The forceps could be set at three different levels of holding strength and to apply the same level of holding for the nerve injury at fixed areas, a black line was marked on the forceps at 5 cm from the end enabling crushing injuries at the fixed area by the strongest holding level. After removing the haemostatic forceps, the first or control group was injected with buffer with 0.05 ml non-modified fibroblast (unit: 5 x 10⁵ cell/50 µl) within 2 mm of nerve injury area using ultra-fine needle (30 gauge). The second and the third experimental groups were injected with gene-modified fibroblasts secreting BMP2 and BMP9, respectively, by the same method before suturing and sterilizing the wounded area.

**Nerve Conduction Test**

Nerve conduction test was performed after anesthetizing the rat with 4% chloral hydrate. The activity-recording electrode was placed on calf muscle to stimulate sciatic notch, the reference electrode was placed on the foot, and the ground electrode was placed between stimulating electrode and the recording electrode. Patch-like electrode was used as the recording electrode and the ground electrode was put under the skin by using a needle electrode. The nerve conduction test was done by using KeyPoint (Dantec, Denmark). Frequency, recording printing rate, and recording sensitivity in this study were set as 2~10,000 Hz, 2 msec/division, 5 mV/division, respectively. Nerve conduction tests were performed every 2 weeks. Measurements were taken from the beginning of the experiment and followed at weeks 2, 4, 6, and 8 afterwards. Latency and amplitude during the tests were measured by the amplitude between the baseline and negative electrode point. 5 rats were selected from each group for the nerve conduction study and 10 measurements were obtained from both. Temperatures of the laboratory and the rats were maintained at 25°C and 30°C, respectively.

**Pathological/Histological Tissue Examination**

Examination of the nerve tissues from rats was carried out to observe natural healing after the injury by examining normal nerves before they were injured from the beginning of the experiment. Four (4) rats (8 nerves) were used to observe regeneration of nerves with cells lacking transgene procedure. 2 rats (4 nerves) were randomly selected from each group and the tissues were examined after 2, 4, and 8 weeks. For the tissue examination, about 2 cm sciatic nerves were exfoliated from the area of crushed injury in anesthetized rats. The changes in nerve tissues were observed under optical microscopy after being fixed with buffered formaldehyde solution and then stained with hematoxylin-eosin (H & E) and modified trichrome (MT) stains.

**Data Analysis**

2, 4, 6 and 8 weeks after the nerve injury, the maximum amplitude of action potential and changes during the latency of compound muscle from each group was analyzed by comparing with reference group, and statistic analysis was carried out by SPSS-PC program. Comparisons between each group were performed by ANOVA and t-test and the level of significance was set at 0.05. The degrees of injury and regeneration were determined by histological data as was interpreted by a pathologist.

**EXAMPLE II - Weight Changes**

Initially, the rats weighed in the range of 400 ± 10 g. Weight changes afterward are shown in Table 2. No difference was observed between each group at week 2, but BMP9 group showed some differences (p<0.05) compared with other groups at weeks 4 and 6. At week 8, there was no statistically significant difference between the groups (p>0.05) (Table 2).

**Table 2. Changes of Body Weight in Experimental Groups**

| | 2^{nd} week | 4^{th} week | 6^{th} week | 8^{th} week |
|---|---|---|---|---|
| Sham | 380.7±29.0 | 365.0±57.7 | 430.0±57.7 | 460.0±46.2 |
| BMP2 | 370.6±24.8 | 368.3±74.7 | 382.5±141.5 | 470.0±40.9 |
| BMP9 | 366.8±34.2 | 442.0±79.8 | 505.0±77.6 | 508.0±60.6 |

**EXAMPLE III - Changes of Latency**

Baseline data measured randomly from each group before the experiment showed latency of 1.44 ± 0.11 msec. Latencies after the trauma showed differences between the control group, BMP2 group and BMP9 group at weeks 2 and 4, but lacked statistical significance (p>0.05). The latencies of BMP2 and BMP9 groups were shortened significantly compared with the control group at week 6 (p<0.05). However, there was no difference in the latencies among the 3 groups at week 8 (p>0.05) (Table 3).

**Table 3. Latency Change in Groups**

| | 2^{nd} week | 4^{th} week | 6^{th} week | 8^{th} week |
|---|---|---|---|---|
| Sham | 1.30±0.11 | 1.29±0.04 | 1.25±0.21 | 1.10±0.0 |
| BMP2 | 1.24±0.14 | 1.25±0.07 | 1.08±0.07* | 1.04±0.04 |
| BMP9 | 1.19±0.12 | 1.23±0.10 | 1.06±0.03* | 1.15±0.07 |

**EXAMPLE IV - Changes of Amplitude**

Baseline data randomly measured from each group before the experiment showed amplitude of 23.9 ± 4.3 mV. Amplitude of BMP9 group increased significantly compared with the control group at weeks 2 and 4 after the trauma (p<0.05). BMP2 and BMP9 groups showed significant differences compared to the control group at week 6 (p<0.05) and significant differences were shown in the order of BMP9, BMP2 and the control groups at week 8 (p<0.05) (Table 4).

**Table 4. Changes of Amplitude**

| | 2^{nd} week | 4^{th} week | 6^{th} week | 8^{th} week |
|---|---|---|---|---|
| Sham | 3.98±1.52 | 6.17±1.27 | 6.33±1.27 | 6.3±1.31 |
| BMP2 | 6.14±1.51 | 7.51±1.29 | 9.00±1.69* | 7.17±0.50* |
| BMP9 | 6.79±1.34* | 9.53±4.47* | 9.47±1.22* | 10.26±2.27* |

**EXAMPLE V - Histological and Pathological Observations**

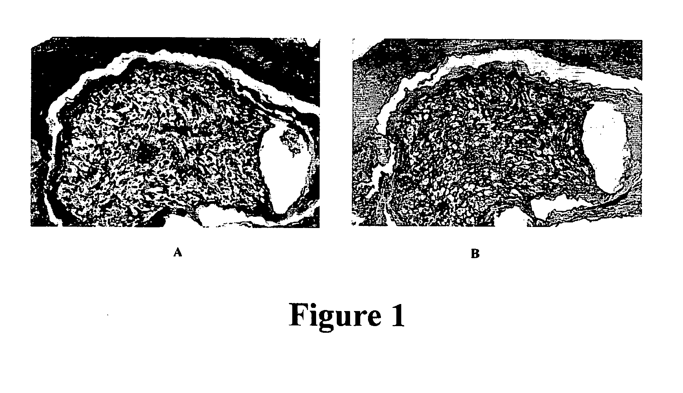

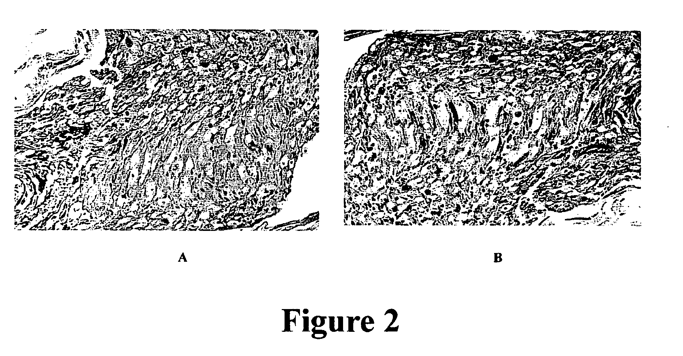

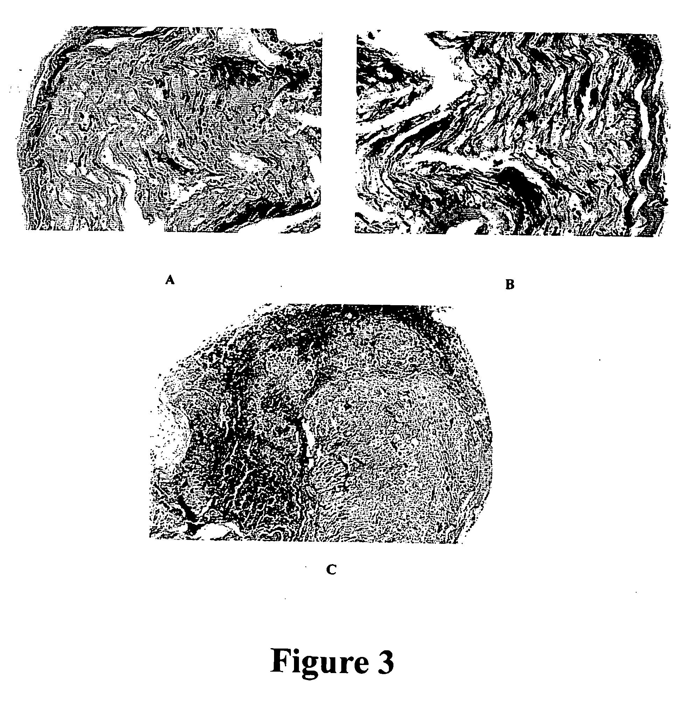

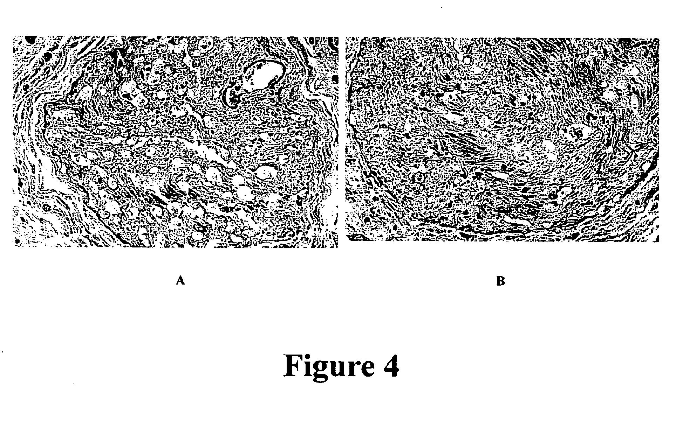

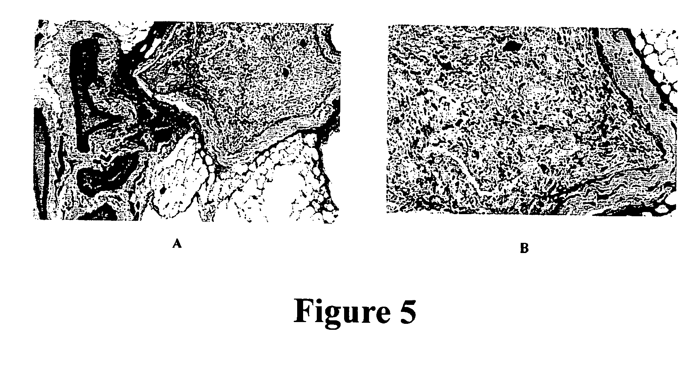

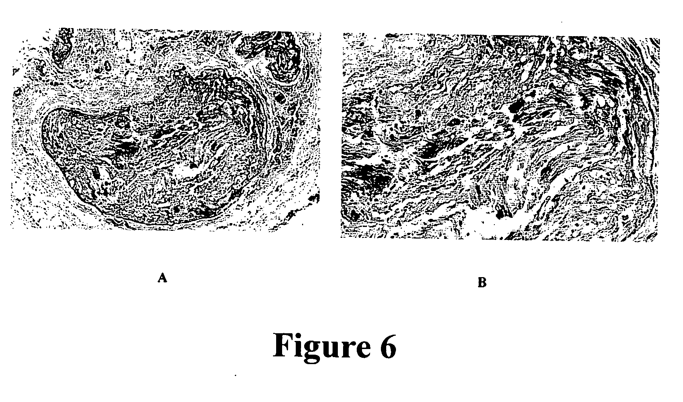

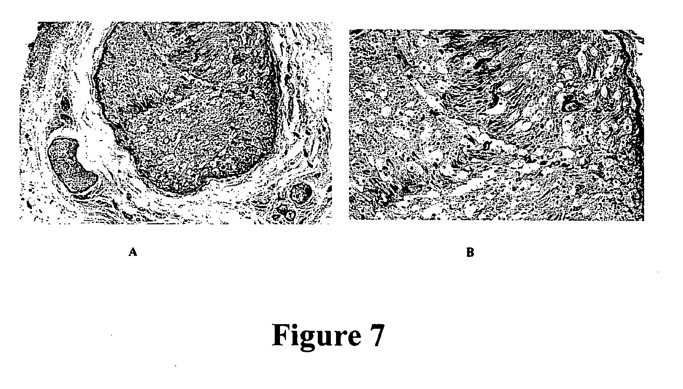

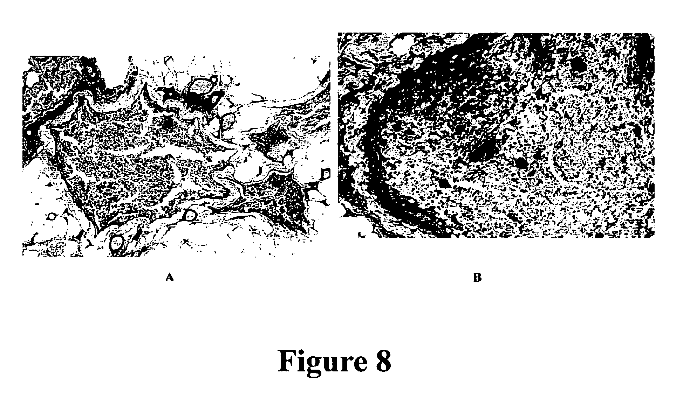

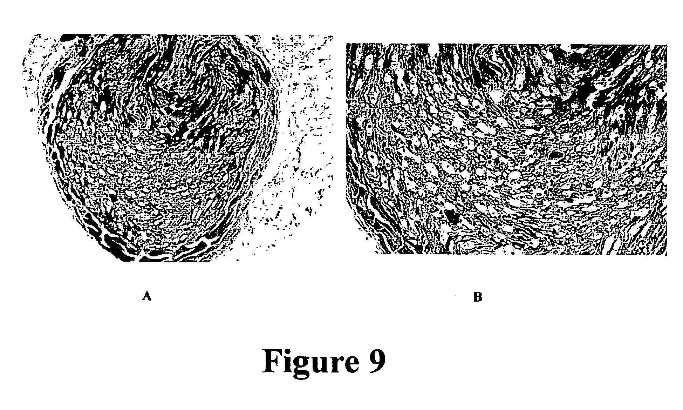

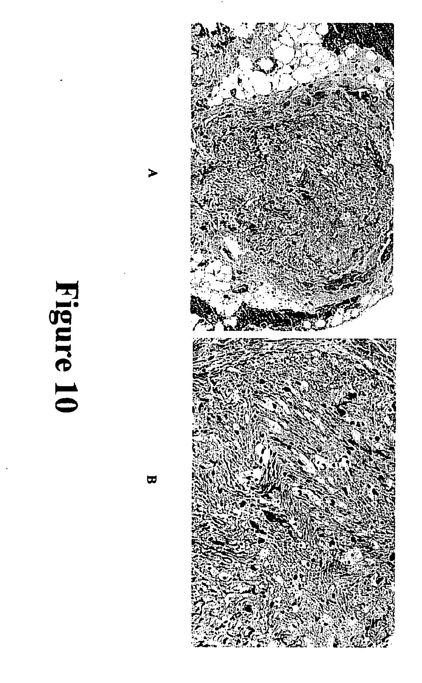

Histological nerve tissue examination of randomly selected rats from each group showed changes similar to the results of the nerve physiology examination as shown in Figure 1 through Figure 10. The control group showed the slowest recovery rate since axons of the nerve tissues were not regenerated and inflammatory reaction remained. The groups transfected with gene-modified fibroblasts secreting BMP2 or BMP9 did not show any significant difference compared to the control group in the early stage, and all groups showed severe pathological indications caused by crushed damage of the nerves such as vacuolar changes and infiltration of inflammatory monocyte, bleeding of epineurium vein at week 2. For the control group, these symptoms persisted throughout 8 weeks. However, for the BMP2 group, the size of inflammation and vacuolar changes were significantly reduced and only half of them showed these symptoms at weeks 4 and 8. For the BMP9 group, these effects were more prominent at weeks 4 and 8, and only one third of them showed loss of axon, and vacuolar changes, and further showed very mild inflammation.

## Claims

1. Use of population of cultured nerve cells transfected *in vitro* with a generated recombinant viral or plasmid vector comprising a DNA sequence encoding a bone morphogenetic protein ("BMP") operatively linked to a promoter, for the preparation of a medicament for regenerating or preventing the degeneration of an injured nerve, wherein the transfected nerve cells are transplanted to an area near the injured nerve, such that expression of the DNA sequence within the area near the injured nerve causes regeneration of the nerve.

2. The use according to claim 1, wherein the transforming growth factor is BMP-2 and BMP-9.

3. The use according to claim 1, wherein the nerve is peripheral nerve.

4. The use according to claim 1, wherein the vector is a viral vector, in particular retroviral vector, adeno-associated viral vector, adenoviral vector, or herpes viral vector.

5. The use of claim 1, wherein said population of cells are stored prior to transplantation.

6. The use of claim 1, wherein said population of transfected cells are stored in 10% DMSO under liquid nitrogen prior to transplantation.

## Patentansprüche

1. Verwendung einer Population kultivierter Nervenzellen, die *in vitro* mit einem erzeugten rekombinanten Virus- oder Plasmid-Vektor transfiziert werden, umfassend eine DNA-Sequenz, die ein knochenmorphogenetisches Protein ("BMP") enkodiert, das zusammenwirkend mit einem Promotor gekoppelt ist, zur Bereitung eines Medikaments zum Regenerieren oder Verhindern der Degenerierung von Nervenzellen, wobei die transfizierten Zellen in einen Bereich nahe eines verletzten Nervs transplantiert werden, so dass die Expression der DNA-Sequenz im Bereich nahe des verletzten Nervs Regeneration des Nervs bewirkt.

2. Verwendung nach Anspruch 1, wobei der transformierende Wachstumsfaktor BMP-2 und BMP-9 ist.

3. Verwendung nach Anspruch 1, wobei der Nerv ein peripherer Nerv ist.

4. Verwendung nach Anspruch 1, wobei der Vektor ein viraler Vektor ist, insbesondere ein retroviraler Vektor, adeno-assoziierter viraler Vektor, adenoviraler Vektor oder Herpes-Virus-Vektor.

5. Verwendung nach Anspruch 1, wobei die Population von Zellen vor der Transplantation gelagert wird.

6. Verwendung nach Anspruch 1, **dadurch gekennzeichnet, dass** die Population von transfizierten Zellen vor der Transplantation in 10% DMSO unter flüssigem Stickstoff gelagert wird.

## Revendications

1. Utilisation d'une population de cellules nerveuses en culture qui sont transfectées *in vitro* avec un vecteur plasmidique ou viral recombinant généré comprenant une séquence d'ADN codant pour une protéine morphogénétique osseuse (« BMP ») en liaison fonctionnelle avec un promoteur, pour la préparation d'un médicament destiné à régénérer ou à prévenir la dégénérescence d'un nerf lésé, dans laquelle les cellules nerveuses transfectées sont transplantées dans une zone à proximité du nerf lésé de sorte que l'expression de la séquence d'ADN au sein de la zone à proximité du nerf lésé entraîne la régénération du nerf.

2. Utilisation selon la revendication 1, dans laquelle le facteur de croissance transformant est la BMP-2 et la BMP-9.

3. Utilisation selon la revendication 1, dans laquelle le nerf est un nerf périphérique.

4. utilisation selon la revendication 1, dans laquelle le vecteur est un vecteur viral, en particulier un vecteur rétroviral, un vecteur viral adéno-associé, un vecteur adénoviral, ou un vecteur dérivé d'un herpésvirus.

5. Utilisation selon la revendication 1, dans laquelle ladite population de cellules est stockée avant la transplantation.

6. Utilisation selon la revendication 1, dans laquelle ladite population de cellules transfectées est stockée dans du DMSO à 10 % dans de l'azote liquide avant la transplantation.
